(19) Europäisches Patentamt

European Patent Office

Office européen des brevets

(11) **EP 1 240 508 B1**

(12) **EUROPÄISCHE PATENTSCHRIFT**

(45) Veröffentlichungstag und Bekanntmachung des Hinweises auf die Patenterteilung:
**18.10.2006 Patentblatt 2006/42**

(21) Anmeldenummer: **00990528.2**

(22) Anmeldetag: **12.12.2000**

(51) Int Cl.:
*G01N 27/72* (2006.01)

(86) Internationale Anmeldenummer:
**PCT/DE2000/004429**

(87) Internationale Veröffentlichungsnummer:
**WO 2001/046682 (28.06.2001 Gazette 2001/26)**

(54) **VERFAHREN UND VORRICHTUNG ZUR IN SITU-ERMITTLUNG DES UMWANDLUNGSGRADS EINER NICHTMAGNETISCHEN PHASE IN EINE FERROMAGNETISCHE PHASE EINES METALLISCHEN WERKSTÜCKS**

METHOD AND DEVICE FOR THE IN SITU DETECTION OF THE DEGREE OF CONVERSION OF A NON-MAGNETIC PHASE IN A FERROMAGNETIC PHASE OF A METALLIC WORKPIECE

PROCEDE ET DISPOSITIF DE DETERMINATION IN SITU DU DEGRE DE CONVERSION D'UNE PHASE AMAGNETIQUE EN UNE PHASE FERROMAGNETIQUE D'UNE PIECE METALLIQUE

(84) Benannte Vertragsstaaten:
**AT BE CH CY DE DK ES FI FR GB GR IE IT LI LU MC NL PT SE TR**

(30) Priorität: **22.12.1999 DE 19962184**

(43) Veröffentlichungstag der Anmeldung:
**18.09.2002 Patentblatt 2002/38**

(73) Patentinhaber: **SIEMENS AKTIENGESELLSCHAFT 80333 München (DE)**

(72) Erfinder:
• **DAALMANS, Gabriel 91315 Höchstadt (DE)**
• **DÖLL, Rüdiger 90408 Nürnberg (DE)**
• **WEINZIERL, Klaus 91052 Erlangen (DE)**

(56) Entgegenhaltungen:
**US-A- 3 449 661**          **US-A- 4 686 471**
**US-A- 4 740 747**

**Beschreibung**

Die Erfindung betrifft ein Verfahren zur in situ-Ermittlung des Umwandlungsgrads einer nichtmagnetischen Phase in eine ferromagnetische Phase eines metallischen Werkstücks nach einer vorangegangenen thermomechanischen Behandlung, insbesondere des $\gamma$-$\alpha$-Phasenumwandlungsgrads eines bandförmigen Stanlwerkstücks, bei dem eine nahe dem Werkstück anzuordnende Messvorrichtung verwendet wird, wobei die Ermittlung anhand der bei Anliegen eines elektromagnetischen Erregerfelds von werkstückseitig hervorgerufenen Wirbelströmen der nichtmagnetischen Phase und von der gegebenenfalls vorliegenden magnetischen Phase erzeugten Antwortfeldern erfolgt, wobei wenigstens ein auf das Werkstück wirkendes elektromagnetisches Erregerfeld mittels wenigstens einer Erregerspule erzeugt wird, und wobei ein aus der Beaufschlagung mit dem Erregerfeld resultierendes Antwortfeld mittels wenigstens einer Detektormessspule, deren vom Abstand der Messvorrichtung zum Werkstück abhängiges Messsignal zur Ermittlung des Umwandlungsgrads dient, gemessen wird.

Die mechanischen Eigenschaften hochwertigen, im Rahmen eines Warmwalzverfahrens hergestellten Stahlblechs hängt neben der Stahlzusammensetzung auch von der Art und Weise der Kühlung des die Walzstraße verlassenden heißen Stahlblechs ab. Beim Kühlen setzt ein Rekristallisierungsprozess ein, welcher abhängig von der jeweiligen Temperatur und der Haltezeit ist, wobei insbesondere die Keimdichte einer sich bildenden Phase zeitabhängig ist. Beim Kühlen des Stahlblechs entsteht neben einer unmagnetischen Phase, der sogenannten $\gamma$-Phase eine ferromagnetische Phase, die sogenannte $\alpha$-Phase. Während des Kühlens werden, abhängig von der Kühlgeschwindigkeit, die Phasen eingefroren, wobei die Phasen nicht im Gleichgewicht stehen.

D.h., abhängig von der Kühlgeschwindigkeit, also der gefahrenen Kühlrampe kann der Phasenanteil variiert werden, wobei hierbei natürlich auch die Stahlzusammensetzung eine Rolle spielt, da die Temperaturen, bei welchen sich eine neue Phase bildet, abhängig von den Stahlzusammensetzungen sind. Die sich bei einer bestimmten Stahlzusammensetzung ergebenden Phasenübergänge sind dem Eisen-Kohlenstoff-Phasendiagramm zu entnehmen.

Die Möglichkeit der Variierung der Phasenanteile mittels eines bestimmten Kühlverfahrens setzt jedoch voraus, dass die Temperatur des Metalls möglichst genau bestimmt werden kann, um zu erkennen, wann die Bildung einer bestimmten Phase einsetzt. Zwar ist es möglich, beispielsweise aufgrund der Messung der Wärmestrahlung des Metalls oder mittels Infrarotabtastung die Temperatur zu bestimmen, jedoch liefern diese Messverfahren nur sehr ungenaue Werte, da die sich auf der Metallbandoberfläche bildende Oxidschicht die Messergebnisse verfälscht. Es ist erforderlich, die Temperatur des unterhalb der Oxidschicht befindlichen Metalls zu messen, was mit den genannten Verfahren aber nicht möglich ist.

Die Bestimmung des Phasenübergangs von der nichtmagnetischen $\gamma$- zur ferromagnetischen $\alpha$-Phase, welcher in Abhängigkeit der konkreten Stahlzusammensetzung bei einer exakt bestimmten, anhand des Eisen-Kohlen-Phasendiagramms ermittelbaren Temperatur erfolgt, kann mittels eines Wirbelstrommessverfahrens erfolgen. Im Rahmen dieses Verfahrens wird ein elektromagnetisches Erregerfeld erzeugt, welches auf das metallische Werkstück einwirkt. Auf dieses Feld reagieren die nichtmagnetische Phase und die ferromagnetische Phase unterschiedlich. In der nichtmagnetischen Phase bewegen sich die Elektronen des metallischen Werkstücks in Abhängigkeit des anliegenden elektromagnetischen Feldes und schirmen das Feld ab. Die E-lektronenbewegung führt zu Wirbelströmen, die wiederum ein Gegenfeld erzeugen, welches das anliegende äußere Erregerfeld schwächt. Es steht diesem entgegen. Die Antwort der nichtmagnetischen Phase führt also zu einer Schwächung des anliegenden elektromagnetischen Feldes.

Die magnetische Phase hingegen reagiert umgekehrt. Aufgrund der Kopplung der magnetischen Momente werden diese in Richtung des anliegenden Erreger- oder Primärfelds ausgerichtet. Das aus der Ausrichtung resultierende Magnetfeld verstärkt das Erregerfeld. Insgesamt liegen also bei Vorliegen der nichtmagnetischen und der ferromagnetischen Phase zwei gegenläufige Prozesse vor.

Wird nun mittels einer geeigneten Detektorspule, die nahe dem metallischen Werkstück angeordnet wird, die Antwort des metallischen Werkstücks gemessen, so wird bei einer oberhalb der Phasenübergangstemperatur zur $\alpha$-Phase liegender Werkstücktemperatur ein geringes insgesamt resultierendes Feld gemessen, was aus der Schwächung des Primärfelds durch die Wirbelstromantwort resultiert. Sobald die Keimbildung der $\alpha$-Phase einsetzt, ändert sich das mittels der Detektorspule messbare resultierende Feld aufgrund des das Primärfeld verstärkenden Feldanteils der magnetischen Phase. Dieser Sprung kann sehr exakt gemessen werden, so dass hieraus eine exakte Temperatur bestimmt werden kann. Aufgrund des zunehmenden Anteils an magnetischer Phase während des Kühlvorgangs ändert sich zunehmend auch das resultierende Feld, so dass aufgrund des erhaltenen Messwerts der temperaturabhängige Umwandlungsgrad bestimmt werden kann.

In der Praxis ist die Bestimmung des Umwandlungsgrades mittels des oben beschriebenen Wirbelstrommessverfahrens allerdings nicht möglich. Dies resultiert aus der Bewegung des Stahlblechs während der Messung. Das aus dem Walzwerk kommende, heiße Stahlblech wird kontinuierlich an der feststehenden Messvorrichtung vorbei zu einer Kühlstrecke hin bewegt. Es liegt jedoch keine homogene Bewegung vor, vielmehr wackelt und rattert das Stahlblech über die Transportrollen der Transportstrecke. Aufgrund dieser inhomogenen Bewegung ergeben sich mehrere Probleme. Die Stärke des Magnetfelds, welches von den Wirbelströmen erzeugt wird, ist abhängig vom Abstand der Werkstückoberfläche zur Detektorspule, es nimmt sehr stark mit zunehmendem Abstand ab. Aufgrund des Wackelns des Blechs bezüglich der ortsfesten Detektorspulen ändert sich in Abhängigkeit der Stahlblechbewegung auch das aufgenommene Messsignal, da das Wirbelstromfeld sich kontinuierlich mit der Bandschwingung ändert. Ferner dringt das elektromagnetische Erregerfeld nicht nur in das Werkstück ein, sondern wird von diesem auch reflektiert. Allein aufgrund des Wackelns erhält man ein das Messsignal beeinflussendes Störsignal.

Ein weiteres Problem liegt in der hohen Metalltemperatur selbst. Die Temperatur für den relevanten Phasenübergang liegt bei ca. 900°C. Die hohe Temperatur führt zu einer gewissen temperaturabhängigen Formänderung der Messkonfigurationen bzw. der Detektorspulen. Sind über die Länge der Kühlstrecke mehrere Spulen verteilt, so können kaum vergleichbare Ergebnisse erhalten werden, da aufgrund der Formänderung sich der Abstand der einzelnen verteilten Spulen relativ zum Metallband ändert. Auch bezogen auf die einzelne Spule können Formänderungen Schwierigkeiten bereiten, da sich die Temperatur des Metallblechs im Bereich der Detektorspulen abhängig von dem momentan verwendeten Abkühlvorgang ändern kann.

Aus US 4,686,471 ist ein Messsystem zur Erfassung des Umwandlungsgrads vorgesehen, das gemäß dem eingangs beschriebenen Verfahren arbeitet, wobei dort eine Erregerspule und zwei Detektorspulen vorgesehen sind. Die Erregerspule und die Detektorspulen liegen alle in einer Ebene, die Detektorspulen sind verschieden weit von der Erregerspule beabstandet.

Der Erfindung liegt damit das Problem zugrunde, ein Verfahren anzugeben, welches die möglichst exakte in situ-Bestimmung des Umwandlungsgrads ermöglicht.

Zur Lösung dieses Problems ist ein Verfahren der eingangs genannten Art mit folgenden Schritten vorgesehen:

- Messen eines aus der Beaufschlagung mit dem Erregerfeld resultierenden Antwortfelds mittels zweier unterschiedlich weit von dem Werkstück beabstandeter Abstandsmessspulen zur Aufnahme zweier in ihrer Größe abstandsabhängiger Messsignale, anhand welcher ein abstandsabhängiger Rechenwert ermittelt wird,
- Ermittlung des Umwandlungsgrads anhand des Messsignals und des Rechenwerts.

Das erfindungsgemäße Verfahren wird im Anspruch 1 definiert. Gemäß dem erfindungsgemäßen Verfahren erfolgt eine tatsächliche Abstandsmessung der Messvorrichtung vom Werkstück. Zu diesem Zweck sind mit besonderem Vorteil zwei Abstandsmessspulen vorgesehen, die unterschiedlich weit vom Werkstück, also dem Stahlband beabstandet sind. Aufgrund der starken Abhängigkeit des Magnetfelds der Wirbelströme vom Abstand der Werkstückoberfläche zur Messvorrichtung erhält man zwei unterschiedliche Signale. Hieraus kann ein Rechenwert ermittelt werden, welcher abhängig vom Abstand ist und ein Maß für den tatsächlichen Abstand gibt. Hierzu kann beispielsweise das Verhältnis der Spulenspannungen ermittelt werden, welches unabhängig von der Größe der Wirbelströme, also der Materialeigenschaft, aber abhängig vom Abstand ist. Als Messsignal wird in diesem Fall also die Spulenspannung verwendet.

Aufgrund des ermittelten abstandsabhängigen Rechenwerts liegt also kontinuierlich ein Maß für den Ist-Abstand vor. Das in der Detektormessspule erhaltene Messsignal, beispielsweise die dort abgreifbare induzierte Spannung, ist ihrerseits abhängig vom Erregerstrom, also dem Erregerfeld sowie einer vom Abstand der Messvorrichtung und dem Umwandlungsgrad abhängigen Funktion. Es gilt ganz allgemein der Zusammenhang

$$V_{\text{Detektorspule}} = I_{\text{Erreger}} \otimes F(h, f\alpha),$$

wobei:

| | |
|---|---|
| $V_{\text{Detektormesspule}}$ | = Induktionsspannung an der Detektormessspule |
| $I_{\text{Brreger}}$ | = Erregerstrom |
| h | = Abstand zwischen Werkstück und Messvorrichtung, |
| $f\alpha$ | = ferromagnetischer Phasenanteil im Blech. |

Ist nun der Abstand zwischen Werkstück und Messvorrichtung bekannt, kann der ferromagnetische Phasenanteil ermittelt werden bzw. auch die Metalltemperatur aufgrund des erfassbaren Phasenanteils bestimmt werden.

Dabei kann erfindungsgemäß die Erregerspule zur Erzeugung eines ersten Erregerfelds, welches ein erstes, von den Abstandsmessspulen zu messendes Antwortfeld erzeugt, mit einer derart hohen Erregerfrequenz betrieben werden, dass das Antwortfeld der nichtmagnetischen Phase größer ist als das der gegebenenfalls vorliegenden ferromagnetischen Phase. Gemäß dieser Erfindungsausgestaltung wird also die Erregerfrequenz so hoch gewählt, dass die Wirbelstromantwort des nichtmagnetischen Teils deutlich größer ist als die des ferromagnetischen Anteils. Dies zu gewährleisten ist sehr vorteilhaft, da das Signal des magnetischen Anteils nach einer anderen Gesetzmäßigkeit mit dem Abstand abfällt als das Signal des nichtmagnetischen Anteils. Die ferromagnetischen Phasenanteile können den hohen Frequenzen nicht vollständig oder überhaupt nicht mehr folgen. Die Abstandsmessspulen messen dann lediglich das Antwortfeld der Wirbelströme. Bevorzugt wird eine Erregerfrequenz ≥ 1 MHz, insbesondere ≥ 10 MHz gewählt.

Gemäß einer weiteren vorteilhaften Erfindungsausgestaltung kann vorgesehen sein, dass die Erregerspule zur Erzeugung eines zweiten Erregerfelds, welches ein zweites, von der Detektormessspule zu messendes Antwortfeld erzeugt, mit einer derart niedrigen Erregerfrequenz betrieben wird, dass das Antwortfeld der nichtmagnetischen Phase kleiner ist als das der gegebenenfalls vorliegenden ferromagnetischen Phase. Die Erregerfrequenz wird also so niedrig gewählt, dass die metallische Wirbelstromantwort, die proportional zur dritten Potenz der Erregerfrequenz ist, im Vergleich zur magnetischen Antwort (die proportional zum Produkt aus Permeabilität und Erregerfrequenz ist) unbedeutend ist. Die Erregerspule sollte hierbei mit einer im kHz-Bereich liegenden Erregerfrequenz beaufschlagt werden. Bevorzugt werden Filter für die jeweiligen Spulen vorgesehen, damit jede nur die für sie relevante Frequenz misst.

Neben dem Phasenumwandlungsgrad stellt die Koerzitivfeldstärke der ferromagnetischen Phase einen interessanten Materialparameter dar. Um diese parallel zum Umwandlungsgrad ermitteln zu können kann erfindungsgemäß vorgesehen sein, dass mittels der oder einer weiteren Erregerspule ein niederfrequentes Magnetfeld erzeugt wird, dessen Feldstärke größer als die Koerzitivfeldstärke des Werkstücks ist, wobei das von dem niederfrequenten Magnetfeld erzeugte Antwortfeld mittels der Detektormessspule gemessen und anhand des erhaltenen Messsignals die Koerzitivfeldstärke des Werkstücks bestimmt wird. Die Feldstärke dieses dritten Erregerfeldes sollte regelbar und größer als das Koerzitivfeld des Werkstücks sein. Die Wirbelstromantwort wird in Abhängigkeit dieses Feldes gemessen, woraus die Koerzitivfeldstärke ermittelt werden kann. Die Koerzitivfeldstärke wird dabei als zusätzliche Messgröße zur Ermittlung des magnetischen Phasenübergangs benutzt. Der Detektormessspule ist zweckmäßigerweise ein Filter vorgeschaltet, der die exakte Messung der unterschiedlichen Antwortfrequenzen, also einerseits des aus der Beaufschlagung mit dem zweiten Erregerfeld resultierenden Antwortfelds und andererseits des aus der Beaufschlagung mit dem dritten Erregerfeld resultierende Antwortfelds, messen kann.

Wenngleich zur Erzeugung der Erregerfelder separate Erregerspulen verwendet werden können, hat es sich als zweckmäßig erwiesen, wenn lediglich eine Erregerspule verwendet wird, die mit unterschiedlichen Erregerfrequenzen beaufschlagt wird. Die Anzahl der verwendeten Erregerspulen richtet sich letztlich nach den konkreten Erregerfrequenzen. Je größer eine Spule ist, desto träger ist sie. Große Spulen werden lediglich zur Erzeugung niederfrequenter Felder verwendet, also beispielsweise dem dritten niederfrequenten Erregerfeld. Zur Erzeugung der ersten beiden Erregerfelder, die höherfrequenter sind, kann eine große Spule bereits zu träge sein, weshalb in diesem Fall zur Erzeugung dieser beiden Felder bevorzugt eine (oder zwei) kleinere, schnellere Spule(n) verwendet wird (werden).

Neben dem erfindungsgemäßen Verfahren betrifft die Erfindung ferner eine Messvorrichtung für eine Messanordnung zur in situ-Ermittlung des Umwandlungsgrads einer nichtmagnetischen Phase in eine ferromagnetische Phase eines metallischen Werkstücks nach einer vorangegangenen Wärmebehandlung, insbesondere des γ-α-Umwandlungsgrads eines bandförmigen Stahlwerkstücks, wobei die Messvorrichtung wenigstens eine Erregerspule zum Erzeugen eines auf das Werkstück einwirkenden elektromagnetischen Erregerfelds und wenigstens eine Detektormessspule zum Messen eines aus der Beaufschlagung des Werkstücks mit dem Erregerfeld resultierenden Antwortfelds umfasst. Die erfindungsgemäße Messvorrichtung zeichnet sich dadurch aus, dass zwei voneinander beabstandete, in unterschiedlichen Abständen bezüglich des Werkstücks positionierbare Abstandsmessspulen zum separaten Messen eines aus der Beaufschlagung des Werkstücks mit einem von der oder einer Erregerspule erzeugten elektromagnetischen Erregerfelds resultierenden Antwortfelds vorgesehen sind, die abstandsabhängige, weiterverarbeitbare Messsignale liefern.

Die erfindungsgemäße Vorrichtung wird im Anspruch 9 definiert. Die beiden Abstandsmessspulen sind bevorzugt hintereinander fluchtend angeordnet. Sie können im Inneren der ringförmigen Erregerspule angeordnet sein. Bei hintereinander fluchtend angeordneten Abstandsmessspulen sollten diese bevorzugt im Zentrum der ringförmigen Erregerspule angeordnet sein, so dass sie insgesamt eine symmetrische Messkonfiguration gibt.

Gemäß einer zweckmäßigen Weiterbildung des Erfindungsgedankens können zwei Detektormessspulen vorgesehen sein. Die zweite Spule ist zur Verbesserung des Signal-Rausch-Verhältnisses von Vorteil, wozu die Messsignale der beiden Spulen kreuzkorreliert werden können. Beide Spulen sollten deswegen gleicher Bauart sein. Die oder die beiden Detektorspulen können ebenfalls im Inneren der ringförmigen Erregerspulen angeordnet sein.

Nach einer vorteilhaften Ausführungsform des Erfindungsgedankens kann eine zweite Erregerspule vorgesehen sein, wobei die eine Erregerspule zum Erzeugen eines ersten Erregerfelds und die andere zum Erzeugen eines zweiten, in seiner Frequenz zum ersten unterschiedlichen Erregerfeldes dient. Das erste erzeugbare Erregerfeld liefert ein erstes werkstückseitig erzeugtes Antwortfeld, welches beispielsweise von den Abstandsspulen gemessen wird. Das zweite, in seiner Frequenz unterschiedliche Erregerfeld liefert ein zweites Antwortfeld, welches eine andere Frequenz als das erste Antwortfeld besitzt, die von der Detektormessspule gemessen werden kann. Entsprechende Filter ermöglichen die frequenzgenaue Messung der Spulen.

Zweckmäßigerweise kann eine weitere Erregerspule vorgesehen sein, die zum Erzeugen eines gegebenenfalls dritten niederfrequenten Erregerfelds dient.

Die oder beide ringförmigen Erregerspulen, die oder beide ringförmigen Detektormessspulen und eine der ringförmigen Abstandsmessspulen sind bevorzugt in einer Ebene liegend angeordnet.

Ferner betrifft die Erfindung eine Messanordnung zur Durchführung des Verfahrens der vorbeschriebenen Art, umfassend eine Messvorrichtung der beschriebenen Art, sowie wenigstens eine Erregungseinheit zum Erregen der Erregerspule(n), wenigstens eine Empfängereinheit zum Empfangen der Messsignale der Detektorspule(n), wenigstens eine Empfängereinheit zum Empfangen der Messsignale der Abstandsmessspulen sowie wenigstens eine Verarbeitungseinheit zum Verarbeiten der Messsignale der Abstandsmessspulen zur Ermittlung des abstandsabhängigen Rechenwerts sowie zur Bestimmung des Umwandlungsgrads anhand des Rechenwerts sowie der Messsignale der Detektormessspule(n). Dabei kann die Erregungseinheit zum Erregen der Erregerspule mit unterschiedlichen Erregungsfrequenzen ausgebildet sein.

Die Verarbeitungseinheit kann erfindungsgemäß zum Ermitteln des Rechenwerts in Form des Verhältnisses der beiden Messsignale, bei denen es sich bevorzugt um die induzierten Spannungen in den Abstandsmessspulen handelt, ausgebildet sein.

In weiterer Erfindungsausgestaltung kann die Verarbeitungseinheit ein Speichermedium umfassen, in welchem eine Vielzahl unterschiedlicher abrufbarer Rechenwerte abgelegt sind, wobei jeder Rechenwert einem unterschiedlichen Paar von Messsignalen zugeordnet ist. Hierdurch kann eine Kalibrierung erreicht werden. Es wird also nicht jedesmal der Rechenwert neu ermittelt, vielmehr wird bei Eingang der Messsignale der dem Signalpaar zugehörige, im Speicher abgelegte Rechenwert erfasst und zur Weiterverarbeitung ausgelesen. Die Kalibrierung kann dadurch erfolgen, dass man die Messvorrichtung neben einem Metallteil mit gleicher Leitfähigkeit wie das herzustellende Stahlblech in unterschiedlichen Abständen positioniert. Zu jedem Abstand werden dann die beiden Abstandsmesssignale aufgenommen und der diesbezügliche Rechenwert ermittelt und signalbezogen abgelegt.

Schließlich kann vorgesehen sein, dass die Verarbeitungseinrichtung zum Ermitteln der Koerzitivfeldstärke des Werkstücks anhand des oder der von den Detektormessspulen gelieferten Messsignale ausgebildet ist.

Weitere Vorteile, Merkmale und Einzelheiten der Erfindung ergeben sich aus den im folgenden beschriebenen Ausführungsbeispielen sowie anhand der Zeichnungen. Dabei zeigen:

Figur 1    eine Prinzipskizze einer erfindungsgemäßen Messanordnung mit einer erfindungsgemäßen Messvorrichtung einer ersten Ausführungsform,

Figur 2    eine erfindungsgemäße Messvorrichtung einer zweiten Ausführungsform, und

Figur 3    eine erfindungsgemäße Messvorrichtung einer dritten Ausführungsform.

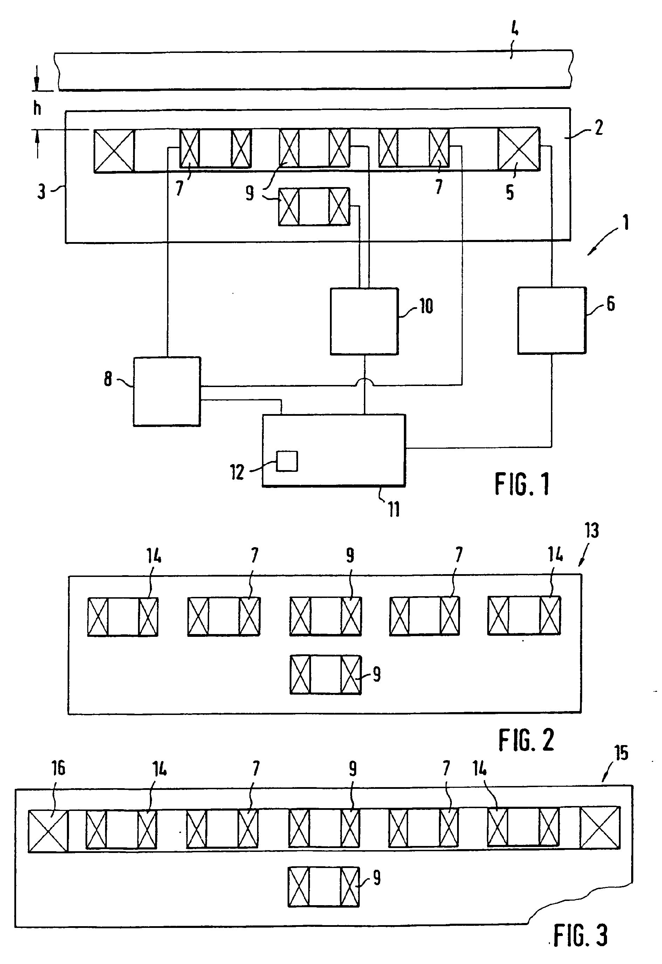

Fig. 1 zeigt eine erfindungsgemäße Messanordnung 1, die eine erfindungsgemäße Messvorrichtung 2 einer ersten Ausführungsform umfasst. Im Gehäuse 3 der Messvorrichtung 2, die nahe einem zu untersuchenden, bandförmigen metallischen Werkstück 4 angeordnet ist, ist eine Erregerspule 5 vorgesehen, bei der es sich um eine größere Ringspule handelt. Die Erregerspule ist mit einer Erregungseinheit 6 gekoppelt, die zum Erregen der Erregerspule 5 dient. Mittels der Erregerspule 5 wird mindestens ein elektromagnetisches Erregerfeld einer bestimmten Frequenz erzeugt, welches auf das Werkstück 4 einwirkt. Das Werkstück 4 besteht abhängig von der Werkstücktemperatur aus einer nichtmagnetischen, metallischen Phase, der sogenannten $\gamma$-Phase und, sofern die Temperatur niedrig genug ist und die Phasenbildung einsetzt oder eingesetzt hat, aus einer ferromagnetischen $\alpha$-Phase. Gegenüber dem beaufschlagten Erregerfeld verhalten sich die beiden Phasen unterschiedlich. In der nichtmagnetischen $\gamma$-Phase werden Elektronen verschoben, es werden Wirbelströme erzeugt, die ihrerseits wieder ein elektromagnetisches Wirbelstrom-Magnetfeld erzeugen, welches dem äußeren Erreger- oder Primärfeld entgegengerichtet ist, dieses also resultierend schwächt. In der gegebenenfalls vorliegenden $\alpha$-Phase werden die magnetischen Momente in Richtung des Erregerfelds ausgerichtet, sie verstärken dieses. Abhängig davon, ob nur nichtmagnetische Phase oder ein Anteil an ferromagnetischer Phase vorliegt ist das resultierende Antwortfeld unterschiedlich. Dieses resultierende Antwortfeld wird mittels zweier Detektormessspulen 7 gemessen. Die Detektormessspulen 7 liefern Messsignale, die einer Empfängereinheit 8 zum Empfang der Messsignale gegeben werden. In dieser Empfängereinheit ist bevorzugt eine Verstärkereinheit integriert, um die Messsignale, bei denen es sich bevorzugt um die abgegriffenen induzierten Spulenspannungen handelt, zu verstärken. Die beiden Messsignale können kreuzkorreliert werden, um das Signal-Rausch-Verhältnis zu verbessern.

Wenngleich die Messvorrichtung 2 bzw. die Spulen in einem bestimmten Abstand h zur Oberfläche des Werkstücks 4 angeordnet sind, so ändert sich dieser Abstand während der Messung laufend aufgrund der transportbedingten inhomogenen Bewegung des bandförmigen Werkstücks 4. Dieses wackelt bezüglich der ortsfesten Messvorrichtung 2, so dass sich kontinuierlich und inhomogen der Abstand h ändert. Da das Magnetfeld der Wirbelströme sehr stark vom Abstand der Werkstückoberfläche zur Messvorrichtung abhängt und mit zunehmendem Abstand sehr stark abnimmt, ist es erforderlich, den Abstand kontinuierlich zu bestimmen, um eine verlässliche Ermittlung des Umwandlungsgrades vornehmen zu können. Zu diesem Zweck sind in der Messvorrichtung 2 zwei Abstandsspulen 9 vorgesehen, die fluchtend hintereinander in unterschiedlichen Abständen zum Werkstück 4 angeordnet sind. Die Abstandsmessspulen 9 messen bevorzugt die Wirbelstromantwort des nichtmagnetischen Phasenanteils, wozu die Erregerfrequenz der Erregerspule 5 entsprechend hoch gewählt wird. Man erhält also zwei unterschiedliche, abstandsabhängige Messsignale. Diese Messsignale werden einer Empfängereinheit 10 gegeben, in welcher sie bevorzugt mittels einer nicht gezeigten Verstärkereinrichtung verstärkt werden. Sämtliche Messsignale sowie die jeweilige Erregerfrequenz werden einer Verarbeitungseinrichtung 11 gegeben, in welcher anhand der gegebenen Signale und Frequenzinformationen der Umwandlungsgrad von der $\gamma$- zur $\alpha$-Phase bestimmt wird. Die Verarbeitungseinrichtung 11 weist ein Speichermedium 12 auf, in dem verschiedene Abstandsmesssignalpaare abgelegt sind, die im Rahmen einer Kalibrierung ermittelt wurden, wobei zu jedem Abstandsmesssignalpaar ein bestimmter Rechenwert abgelegt ist. Die tatsächliche Bestimmung des Umwandlungsgrades erfolgt nun anhand des signalabhängigen, abstandsabhängigen Rechenwerts, welcher anhand der gegebenen Abstandsmesssignale aus dem Speichermedium ausgelesen werden kann, sowie anhand der Signale der Detektormessspulen. Das Ermittlungsergebnis kann von der Verarbeitungseinrichtung 11 an ein beliebiges Ausgabemedium ausgegeben werden.

Die Erregungseinheit 6 ist zum gleichzeitigen Erregen der Erregerspule 5 mit unterschiedlichen Erregerfrequenzen ausgebildet. Zum einen wird die Erregerspule 5 mit einer hohen Erregerfrequenz betrieben, so dass ein erstes hochfrequentes Erregerfeld erzeugt wird, dessen Frequenz so hoch ist, dass die Wirbelstromantwort des nichtmagnetischen Phasenteils deutlich größer ist als die des ferromagnetischen Phasenanteils. Das hieraus resultierende Antwortfeld wird von den beiden Abstandsmessspulen 9 gemessen. Hierzu sind entsprechende Filter vorgesehen, die gewährleisten, dass die Abstandsmessspulen 9 ausschließlich diesen Frequenzbereich erfassen.

Ferner wird die Erregerspule 5 mit einer niederfrequenteren Frequenz betrieben, um ein zweites Erregerfeld zu erzeugen, dessen Frequenz wiederum so niedrig gewählt ist, dass die Wirbelstromantwort der metallischen Phase im Vergleich zur Antwort der ferromagnetischen Phase gering bzw. vernachlässigbar ist. Das hieraus resultierende niederfrequente Antwortfeld wird mittels der beiden Detektorspulen gemessen. Auch hier sind entsprechende Filter vorgesehen. Schließlich kann die Erregerspule 5 mit einer dritten, niederfrequenten Frequenz erregt werden, um ein drittes niederfrequentes Magnetfeld zu erzeugen. Die Feldstärke dieses Magnetfelds sollte regelbar und größer als das Koerzitivfeld des Metalls sein. Die niederfrequente Wirbelstromantwort auf dieses niederfrequente Erregerfeld wird ebenfalls mittels der Detektorspule 7 gemessen. Die Verarbeitungseinrichtung 11 ist in der Lage, aus diesen niederfrequenten Messsignalen die Koerzitivfeldstärke des Metalls zu ermitteln.

Fig. 2 zeigt eine weitere Ausführungsform einer erfindungsgemäßen Messvorrichtung 13. Diese weist, wie auch die Messvorrichtung 2, zwei Abstandsmessspulen 9 sowie zwei Detektormessspulen 7 auf. Jedoch sind zwei separate Erregerspulen 14 vorgesehen, von denen die eine zur Erzeugung der beiden oben beschriebenen ersten Erregerfelder dient, die andere Erregerspule dient zur Erzeugung des sehr niederfrequenten dritten Feldes, welches zur Ermittlung der Koerzitivfeldstärke dient. Mit diesem niederfrequenten Feld wird quasi die Hystereseschleife im Bereich der Koerzitivfeldstärke abgefahren. Diese Ausgestaltung ist dahingehend von Vorteil, als die zur Erzeugung dieses sehr niederfrequenten Feldes erforderliche Erregerspule relativ langsam und träge ist und die doch deutlich höhere Frequenzen, die zur Erzeugung der beiden genannten höherfrequenteren Felder erforderlich sind, nicht erzeugen kann.

Schließlich zeigt Fig. 3 eine dritte Ausführungsform einer erfindungsgemäßen Messvorrichtung 15. In dieser sind wiederum die beiden Abstandsmessspulen 9 sowie die beiden Detektormessspulen 7 vorgesehen. Auch sind zwei Erregerspulen 14 vorgesehen, von denen die eine zur Erzeugung des ersten hochfrequenten, die andere zur Erzeugung des zweiten niederfrequenteren Erregerfeldes dient. Ferner ist eine dritte Erregerspule 16 vorgesehen, die zur Erzeugung des niederfrequenten, der Bestimmung der Koerzitivfeldstärke dienenden Erregerfeldes dient.

Wie den Figuren zu entnehmen ist, sind sämtliche Spulen mit Ausnahme der einen Abstandsmessspule 9 in einer Ebene angeordnet, so dass sie also alle um den gleichen Abstand von der Werkstückoberfläche beabstandet sind.

**Patentansprüche**

1. Verfahren zur in situ-Ermittlung des Umwandlungsgrads einer nichtmagnetischen Phase in eine ferromagnetische Phase eines metallischen Werkstücks nach einer vorangegangenen thermomechanischen Behandlung, insbesondere des $\gamma$-$\alpha$-Phasenumwandlungsgrads eines bandförmigen Stahlwerkstücks, bei dem eine nahe dem Werkstück anzuordnende Messvorrichtung verwendet wird, wobei die Ermittlung anhand der bei Anliegen eines elektromagnetischen Erregerfelds von werkstückseitig hervorgerufenen Wirbelströmen der nichtmagnetischen Phase und von der gegebenenfalls vorliegenden magnetischen Phase erzeugten Antwortfeldern erfolgt, wobei wenigstens ein auf das Werkstück wirkendes elektromagnetisches Erregerfeld mittels wenigstens einer Erregerspule erzeugt wird, und wobei ein aus der Beaufschlagung mit dem Erregerfeld resultierendes Antwortfeld mittels wenigstens einer Detektormessspule, deren vom Abstand der Messvorrichtung zum Werkstück abhängiges Messsignal zur Ermittlung des Umwandlungsgrads dient, gemessen wird, **gekennzeichnet durch** folgende Schritte:

   - Messen eines aus der Beaufschlagung mit dem Erregerfeld resultierenden Antwortfelds mittels zweier unterschiedlich weit von dem Werkstück beabstandeter Abstandsmessspulen zur Aufnahme zweier in ihrer Größe abstandsabhängiger Messsignale, anhand welcher ein abstandsabhängigen Rechenwert ermittelt wird,
   - Ermittlung des Umwandlungsgrads anhand des Messsignals der Detektormessspule und des Rechenwerts.

**2.** Verfahren nach Anspruch 1, **dadurch gekennzeichnet, dass** als Rechenwert der sich aus den beiden Messsignalen ergebende Verhältniswert verwendet wird.

**3.** Verfahren nach Anspruch 1 oder 2, **dadurch gekennzeichnet, dass** die Erregerspule zur Erzeugung eines ersten Erregerfelds, welches ein erstes, von den Abstandsmessspulen zu messendes Antwortfeld erzeugt, mit einer derart hohen Erregerfrequenz betrieben wird, dass das Antwortfeld der nichtmagnetischen Phase größer ist als das der gegebenenfalls vorliegenden ferromagnetischen Phase.

**4.** Verfahren nach Anspruch 3, **dadurch gekennzeichnet, dass** die Erregerfrequenz $\geq 1$ MHz, insbesondere $\geq 10$ MHz ist.

**5.** Verfahren nach einem der vorangehenden Ansprüche, **dadurch gekennzeichnet, dass** die Erregerspule zur Erzeugung eines zweiten Erregerfelds, welches ein zweites, von der Detektormessspule zu messendes Antwortfeld erzeugt, mit einer derart niedrigen Erregerfrequenz betrieben wird, dass das Antwortfeld der nichtmagnetischen Phase kleiner ist als das der gegebenenfalls vorliegenden ferromagnetischen Phase.

**6.** Verfahren nach Anspruch 5, **dadurch gekennzeichnet, dass** die Erregerspule mit einer im kHz-Bereich liegenden Erregerfrequenz beaufschlagt wird.

**7.** Verfahren nach einem der vorangehenden Ansprüche, **dadurch gekennzeichnet, dass** mittels der oder einer weiteren Erregerspule ein niederfrequentes Magnetfeld erzeugt wird, dessen Feldstärke größer als die Koerzitivfeldstärke des Werkstücks ist, wobei das von dem niederfrequenten Magnetfeld erzeugte Antwortfeld mittels der Detektormessspule gemessen und anhand des erhaltenen Messsignals die Koerzitivfeldstärke des Werkstücks bestimmt wird.

**8.** Verfahren nach einem der vorangehenden Ansprüchen, **dadurch gekennzeichnet, dass** eine Erregerspule verwendet wird, die mit unterschiedlichen Erregerfrequenzen beaufschlagt wird.

**9.** Messvorrichtung für eine Messanordnung zur in situ-Ermittlung des Umwandlungsgrads einer nichtmagnetischen Phase in eine ferromagnetische Phase eines metallischen Werkstücks nach einer vorangegangenen Wärmebehandlung, insbesondere des $\gamma$-$\alpha$-Phasenumwandlungsgrads eines bandförmigen Stahlwerkstücks, wobei die Messvorrichtung wenigstens eine Erregerspule zum Erzeugen eines auf das Werkstück einwirkenden elektromagnetischen Erregerfelds und eine Detektormessspule zum Messen eines aus der Beaufschlagung des Werkstücks mit dem Erregerfeld resultierenden Antwortfelds umfasst, **dadurch gekennzeichnet, dass** zwei voneinander beabstandete, in unterschiedlichen Abständen bezüglich des Werkstücks (4) positionierbare Abstandsmessspulen (9) zum separaten Messen eines aus der Beaufschlagung des Werkstücks (4) mit einem von der oder einer Erregerspule (5, 14) erzeugten elektromagnetischen Erregerfelds resultierenden Antwortfelds vorgesehen sind, die abstandsabhängige, weiterverarbeitbare Messsignale liefern.

**10.** Messvorrichtung nach Anspruch 9, **dadurch gekennzeichnet, dass** die beiden Abstandsmessspulen (9) hintereinander fluchtend angeordnet sind.

**11.** Messvorrichtung nach Anspruch 9 oder 10, **dadurch gekennzeichnet, dass** die beiden Abstandsmessspulen (9) im Inneren der ringförmigen Erregerspule (5) angeordnet sind.

**12.** Messvorrichtung nach Anspruch 11, **dadurch gekennzeichnet, dass** die beiden hintereinander fluchtend angeordneten Abstandsmessspulen (9) in Zentrum der ringförmigen Erregerspule (5) angeordnet sind.

**13.** Messvorrichtung nach einem der Ansprüche 9 bis 12, **dadurch gekennzeichnet, dass** zwei Detektormessspulen (7) vorgesehen sind.

**14.** Messvorrichtung nach einem der Ansprüche 9 bis 13, **dadurch gekennzeichnet, dass** die oder beide Detektormesspulen (7) im Inneren der ringförmigen Erregerspule (5) angeordnet sind.

**15.** Messvorrichtung nach einem der Ansprüche 9 bis 14, **dadurch gekennzeichnet, dass** eine zweite Erregermessspule (14) vorgesehen ist, wobei die eine Erregermessspule zum Erzeugen eines ersten Erregerfelds und die andere zum Erzeugen eines zweiten, in seiner Frequenz zum ersten unterschiedlichen Erregerfelds dient.

16. Messvorrichtung nach einem der Ansprüche 9 bis 15, **dadurch gekennzeichnet, dass** eine weitere Erregerspule (16) vorgesehen ist, die zum Erzeugen eines gegebenenfalls dritten niederfrequenten Erregerfelds dient.

17. Messvorrichtung nach einem der Ansprüche 9 bis 16, **dadurch gekennzeichnet, dass** die ringförmige(n) Erregerspule(n) (5, 14, 16), die oder beide ringförmigen Detektormessspulen (7) und eine der ringförmigen Abstandsmessspulen (9) in einer Ebene liegend angeordnet sind.

18. Messanordnung zur Durchführung des Verfahrens nach einem der Ansprüche 1 bis 8, umfassend eine Messvorrichtung (2, 13, 15) nach einem der Ansprüche 9 bis 17 sowie eine Erregungseinheit (6) zum Erregen der Erregerspule(n) (5, 14, 16), wenigstens eine Empfängereinheit (8) zum Empfangen der Messsignale der Detektorspule(n) (7), wenigstens eine Empfängereinheit (10) zum Empfangen der Messsignale der Abstandsmessspulen (9) sowie wenigstens eine Verarbeitungseinheit (11)zum Verarbeiten der Messsignale der Abstandsmessspulen (9) zur Ermittlung des abstandsabhängigen Rechenwerts sowie zur Bestimmung des Umwandlungsgrads anhand des Rechenwerts sowie des Messsignals der Detektorspule(n) (7).

19. Messanordnung nach Anspruch 18, **dadurch gekennzeichnet, dass** die Erregungseinheit (6) zum Erregen der Erregungsspule(n) (5, 14, 16) mit unterschiedlichen Erregungsfrequenzen ausgebildet ist.

20. Messanordnung nach Anspruch 18 oder 19, **dadurch gekennzeichnet, dass** die Verarbeitungseinheit (11) zum Ermitteln des Rechenwerts in Form des Verhältnisses der beiden Messsignale ausgebildet ist.

21. Messanordnung nach einem der Ansprüche 18 bis 20, **dadurch gekennzeichnet, dass** in einem Speichermedium (12) der Verarbeitungseinheit (11) eine Vielzahl unterschiedlicher abrufbarer Rechenwerte abgelegt sind, wobei jeder Rechenwert einem unterschiedlichen Paar von Messsignalen zugeordnet ist.

22. Messanordnung nach einem der Ansprüche 18 bis 21, **dadurch gekennzeichnet, dass** die Verarbeitungseinrichtung (11) zum Ermitteln der Koerzitivfeldstärke des Werkstücks anhand des oder der von den Detektormessspulen (7) gelieferten Messsignals ausgebildet ist.

**Claims**

1. Method for the in situ detection of the degree of transformation of a nonmagnetic phase into a ferromagnetic phase of a metallic workpiece after a preceding thermomechanical treatment, in particular the degree of $\gamma$-$\alpha$ phase transformation of a steel workpiece in strip form, in which a measuring device, which is to be arranged close to the workpiece, is used, the detection taking place on the basis of the eddy currents of the nonmagnetic phase, which are caused from the workpiece when an electromagnetic excitation field is applied, and of response fields which are generated by the magnetic phase which may be present, at least one electromagnetic excitation field which acts on the workpiece being generated by means of at least one excitation coil, and a response field which results from the application of the excitation field being measured by means of at least one detector measurement coil, the measurement signal from which, which is dependent on the distance from the measuring device to the workpiece, being used to detect the degree of transformation, **characterized by** the following steps:

   — measuring a response field, which results from the application of the excitation field, by means of two distance-measuring coils, which are at different distances from the workpiece, for receiving two measurement signals, the level of which is dependent on the distance and which are used to determine a distance-dependent calculation value,
   — detection of the degree of transformation on the basis of the measurement signal from the detector measurement coil and the calculation value.

2. Method according to Claim 1, **characterized in that** the ratio which results from the two measurement signals is used as the calculation value.

3. Method according to Claim 1 or 2, **characterized in that** the excitation coil, in order to generate a first excitation field which generates a first response field which is to be measured by the distance-measuring coils, is operated with an excitation frequency which is so high that the response field of the nonmagnetic phase is greater than that of the ferromagnetic phase which may be present.

**4.** Method according to Claim 3, **characterized in that** the excitation frequency is ≥ 1 MHz, in particular ≥ 10 MHz.

**5.** Method according to one of the preceding claims, **characterized in that** the excitation coil, in order to generate a second excitation field, which generates a second response field which is to be measured by the detector measurement coil, is operated with an excitation frequency which is so low that the response field of the nonmagnetic phase is lower than that of the ferromagnetic phase which may be present.

**6.** Method according to Claim 5, **characterized in that** the excitation coil is acted on by an excitation frequency which is in the kHz range.

**7.** Method according to one of the preceding claims, **characterized in that** a low-frequency magnetic field, the field strength of which is greater than the coercive field strength of the workpiece, is generated by means of the or a further excitation coil, the response field which is generated by the low-frequency magnetic field being measured by means of the detector measurement coil and the coercive field strength of the workpiece being determined on the basis of the measurement signal obtained.

**8.** Method according to one of the preceding claims, **characterized in that** an excitation coil which is acted on by different excitation frequencies is used.

**9.** Measuring device for a measuring arrangement for the in situ detection of the degree of transformation of a nonmagnetic phase into a ferromagnetic phase of a metallic workpiece after a preceding heat treatment, in particular the degree of γ-α phase transformation of a steel workpiece in strip form, the measuring device comprising at least one excitation coil for generating an electromagnetic excitation field which acts on the workpiece, and a detector measurement coil for measuring a response field which results from the application of the excitation field to the workpiece, **characterized in that** two distance-measuring coils (9), which are spaced apart from one another and can be positioned at different distances with respect to the workpiece (4), are provided for separately measuring a response field which results from the application of an electromagnetic excitation field, generated by the or one excitation coil (5, 14) to the workpiece (4), these distance-measuring coils supplying measurement signals which are dependent on distance and can be processed further.

**10.** Measuring device according to Claim 9, **characterized in that** the two distance-measuring coils (9) are arranged aligned one behind the other.

**11.** Measuring device according to Claim 9 or 10, **characterized in that** the two distance-measuring coils (9) are arranged in the interior of the annular excitation coil (5).

**12.** Measuring device according to Claim 11, **characterized in that** the two distance-measuring coils (9), which are arranged aligned one behind the other, are arranged in the center of the annular excitation coil (5).

**13.** Measuring device according to one of Claims 9 to 12, **characterized in that** two detector measurement coils (7) are provided.

**14.** Measuring device according to one of Claims 9 to 13, **characterized in that** the or both detector measurement coils (7) are arranged in the interior of the annular excitation coil (5) .

**15.** Measuring device according to one of Claims 9 to 14, **characterized in that** a second excitation measurement coil (14) is provided, one excitation measurement coil being used to generate a first excitation field and the other excitation measurement coil being used to generate a second excitation field, the frequency of which differs from that of the first excitation measurement coil.

**16.** Measuring device according to one of Claims 9 to 15, **characterized in that** there is a further excitation coil (16), which is used to generate a third, low-frequency excitation field if necessary.

**17.** Measuring device according to one of Claims 9 to 16, **characterized in that** the annular excitation coil(s) (5, 14, 16), the or both annular detector measurement coils (7) and one of the annular distance-measuring coils (9) are arranged such that they lie in one plane.

**18.** Measuring arrangement for carrying out Method according to one of Claims 1 to 8, comprising a measuring device (2, 13, 15) according to one of Claims 9 to 17 and an excitation unit (6) for exciting the excitation coil(s) (5, 14, 16), at least one receiver unit (8) for receiving the measurement signals from the detector coil(s) (7), at least one receiver unit (10) for receiving the measurement signals from the distance-measuring coils (9), and at least one processing unit (11) for processing the measurement signals from the distance-measuring coils (9) in order to determine the distance-dependent calculation value and to determine the degree of transformation on the basis of the calculation value and the measurement signal from the detector coil (s) (7).

19. Measuring arrangement as claimed in Claim 18, **characterized in that** the excitation unit (6) for exciting the excitation coil(s) (5, 14, 16) is designed with different excitation frequencies.

20. The measuring arrangement as claimed in Claim 18 or 19, **characterized in that** the processing unit (11) is designed to determine the calculation value in the form of the ratio of the two measurement signals.

21. The measuring arrangement as claimed in one of Claims 18 to 20, **characterized in that** a multiplicity of different retrievable calculation values are stored in a storage medium (12) of the processing unit (11), each calculation value being assigned to a different pair of measurement signals.

22. The measuring arrangement as claimed in one of Claims 18 to 21, **characterized in that** the processing device (11) is designed to determine the coercive field strength of the workpiece on the basis of the measurement signal (s) supplied by the detector measurement coils (7).


**Revendications**

1. Procédé de détermination in situ du degré de conversion d'une phase amagnétique en une phase ferromagnétique d'une pièce métallique après un traitement thermomécanique ayant eu lieu auparavant, notamment du degré de conversion de phases $\gamma$-$\alpha$ d'une pièce en acier en forme de feuillard, dans lequel on utilise un dispositif de mesure disposé près de la pièce, la détermination s'effectuant au moyen des champs de réponse produits lors de l'application d'un champ électromagnétique d'excitation par des courants de Foucault, provoqués du côté de la pièce, de la phase non magnétique et de la phase magnétique éventuellement présente, au moins un champ électromagnétique d'excitation agissant sur la pièce étant produit au moyen d'au moins une bobine d'excitation et un champ de réponse provenant de la soumission au champ d'excitation étant mesuré au moyen d'une bobine de détecteur dont le signal de mesure, qui dépend de la distance du dispositif de mesure à la pièce, sert à la détermination du degré de conversion, **caractérisé par** les stades suivants :

   - on mesure un champ de réponse provenant de la soumission au champ d'excitation au moyen de deux bobines de mesure de distance éloignées différemment de la pièce pour enregistrer deux signaux de mesure d'intensités différentes en fonction de la distance au moyen desquels on détermine un valeur de calcul en fonction de la distance,
   - on détermine le degré de conversion au moyen du signal de mesure de la bobine de détecteur et de la valeur de calcul.

2. Procédé suivant la revendication 1, **caractérisé en ce que** l'on utilise comme valeur de calcul la valeur du rapport des deux signaux de mesure.

3. Procédé suivant la revendication 1 ou 2, **caractérisé en ce que** l'on fait fonctionner la bobine d'excitation pour la production d'un premier champ d'excitation qui produit un premier champ de réponse à mesurer par les bobines de mesure de distance à une fréquence d'excitation si haute que le champ de réponse de la phase amagnétique est plus grand que celui de la phase ferromagnétique éventuellement présente.

4. Procédé suivant la revendication 3, **caractérisé en ce que** la fréquence d'excitation est $\geq$ 1 MHz, notamment $\geq$ 10 MHz.

5. Procédé suivant l'une des revendications précédentes, **caractérisé en ce que** l'on fait fonctionner la bobine d'excitation pour la production d'un deuxième champ d'excitation qui produit un deuxième champ de réponse à mesurer par la bobine de mesure formant détecteur à une fréquence d'excitation si basse que le champ de réponse de la phase amagnétique est plus petit que celui de la phase ferromagnétique éventuellement présente.

**6.** Procédé suivant la revendication 5, **caractérisé en ce que** l'on alimente la bobine d'excitation à une fréquence d'excitation dans le domaine du kHz.

**7.** Procédé suivant l'une des revendications précédentes, **caractérisé en ce que** l'on produit au moyen de la bobine d'excitation et d'une autre bobine d'excitation un champ magnétique à basse fréquence dont l'intensité est plus grande que l'intensité du champ coercitif de la pièce, le champ de réponse produit par le champ magnétique à basse fréquence étant mesuré au moyen de la bobine de mesure formant détecteur et l'intensité du champ coercitif de la pièce étant déterminée au moyen du signal de mesure obtenu.

**8.** Procédé suivant l'une des revendications précédentes, **caractérisé en ce que** l'on utilise une bobine d'excitation qui est alimentée à des fréquences d'excitation différentes.

**9.** Dispositif de mesure pour un agencement de mesure pour la détermination in situ du degré de conversion d'une phase amagnétique en une phase ferromagnétique d'une pièce métallique après un traitement thermique précédent, notamment du degré de conversion de phases $\gamma$-$\alpha$ d'une pièce en acier en forme de feuillard, le dispositif de mesure ayant au moins une bobine d'excitation pour produire un champ électromagnétique d'excitation agissant sur la pièce et une bobine de mesure formant détecteur pour mesurer un champ de réponse provenant de la soumission de la pièce au champ d'excitation, **caractérisé en ce qu'**il est prévu deux bobines (9) de mesure de distance à distance l'une de l'autre et pouvant être mises en position à des distances différentes de la pièce (4) pour mesurer séparément un champ de réponse provenant d'un champ électromagnétique d'excitation produit par la bobine ou par l'une des bobines (5, 14) d'excitation, les bobines (9) fournissant des signaux de mesure qui dépendent de la distance et qui peuvent être traités davantage.

**10.** Dispositif de mesure suivant la revendication 9, **caractérisé en ce que** les deux bobines (9) de mesure de distance sont disposées en alignement l'une derrière l'autre.

**11.** Dispositif de mesure suivant la revendication 9 ou 10, **caractérisé en ce que** les deux bobines (9) de mesure de distance sont disposées à l'intérieur de la bobine (9) annulaire d'excitation.

**12.** Dispositif de mesure suivant la revendication 11, **caractérisé en ce que** les deux bobines (9) disposées en alignement l'une derrière l'autre sont disposées au centre de la bobine (5) annulaire d'excitation.

**13.** Dispositif de mesure suivant l'une des revendications 9 à 12, **caractérisé en ce qu'**il est prévu deux bobines (7) de mesure à détecteur.

**14.** Dispositif de mesure suivant l'une des revendications 9 à 13, **caractérisé en ce que** la ou les deux bobines (7) de mesure à détecteur sont disposées à l'intérieur de la bobine (5) annulaire d'excitation.

**15.** Dispositif de mesure suivant l'une des revendications 9 à 14, **caractérisé en ce qu'**il est prévu une deuxième bobine (14) de mesure d'excitation, la première bobine de mesure d'excitation servant à produire un premier champ d'excitation et l'autre à produire un deuxième champ d'excitation différent par sa fréquence du premier.

**16.** Dispositif de mesure suivant l'une des revendications 9 à 15, **caractérisé en ce qu'**il est prévu une autre bobine (16) d'excitation qui sert à produire, le cas échéant, un troisième champ d'excitation en basse fréquence.

**17.** Dispositif de mesure suivant l'une des revendications 9 à 16, **caractérisé en ce que** la ou les bobine(s) (5, 14, 16) annulaire(s) d'excitation, la ou les deux bobines (7) annulaires de mesure à détecteur et l'une des bobines (9) annulaires de mesure de distance sont disposées dans un plan.

**18.** Agencement de mesure pour la mise en oeuvre du procédé suivant l'une des revendications 1 à 8, comprenant un dispositif (2, 13, 15) de mesure suivant l'une des revendications 9 à 17 ainsi qu'une unité (6) d'excitation de la ou des bobine(s) (5, 14, 16) d'excitation, au moins une unité (8) de réception des signaux de mesure de la ou des bobine(s) (7) à détecteur, au moins une unité (10) de réception des signaux de mesure des bobines (9) de mesure de distance ainsi qu'au moins une unité (11) de traitement des signaux de mesure des bobines (9) de mesure de distance pour déterminer la valeur de calcul en fonction de la distance ainsi que pour déterminer le degré de conversion moyen de la valeur de calcul ainsi que du signal de mesure de la ou des bobine(s) (7) à détecteur.

**19.** Agencement de mesure suivant la revendication 18, **caractérisé en ce que** l'unité (6) d'excitation est constituée pour exciter la ou les bobine(s) (5, 14, 16) d'excitation à des fréquences d'excitation différentes.

20. Agencement de mesure suivant la revendication 18 ou 19, **caractérisé en ce que** l'unité (11) de traitement est constituée pour déterminer la valeur du calcul sous la forme du rapport des deux signaux de mesure.

21. Agencement de mesure suivant l'une des revendications 18 à 20, **caractérisé en ce qu'**une pluralité de valeurs de calcul différentes qui peuvent être appelées est mémorisée dans un support (12) de mémoire de l'unité (11) de traitement, chaque valeur de calcul étant associée à une paire différente de signaux de mesure.

22. Agencement de mesure suivant l'une des revendications 18 à 21, **caractérisé en ce que** le dispositif (11) de traitement est constitué pour déterminer l'intensité du champ coercitif de la pièce au moyen du signal ou des signaux de mesure fournis par les bobines (7) de mesure à détecteur.

FIG. 1

FIG. 2

FIG. 3